# EUROPEAN PATENT APPLICATION

(11) **EP 4 374 798 A1**
(43) Date of publication of application: **29.05.2024**
(21) Application number: 22208743.9
(22) Date of filing: 22.11.2022
(51) Int. Cl.: A61B 17/15, A61B 17/14, A61B 34/20, A61B 90/00

(54) **SYSTEM FOR ASSISTING A SURGEON DURING THE PERFORMANCE OF A CUT IN A BONE OF A PATIENT**

(71) Applicant: Hvidovre Hospital, 2650 Hvidovre (DK); Københavns Universitet, 1165 Copenhagen K (DK)
(72) Inventor: Jensen, Christian Bredgaard, 2650 Hvidovre (DK); Troelsen, Anders, 1165 Copenhagen K (DK)
(74) Representative: Plougmann Vingtoft a/s

(57) **Abstract**

The invention relates to a system for assisting a surgeon during the performance of a cut, such as a sagittal cut, in a bone (2) of a patient. It comprises a first sensor (8) to be arranged on an associated cutting guide (3) placed on the bone (2), the cutting guide defining a desired cutting orientation. It also comprises a second sensor (9) to be arranged on an associated bone-saw (1). The first and second sensors measure the spatial orientation of the cutting guide and a blade (5) of the bone-saw, respectively, and transmit output signals (10,11) representative of the spatial orientations to a receiver (12). Based on the received first and second output signals, the receiver determines a difference (β) between the spatial orientations of the blade and the cutting guide and present an indication of the difference to the surgeon on at least one angle conformity indicator (13).

## Description

### FIELD OF THE INVENTION

The present invention relates to a system for assisting a surgeon during the performance of a bone surgery, such as an arthroplasty surgery. In particular, it relates to a system which is suitable for guidance of the performance of a cut during the preparation of the bone when performing such a surgery, e.g. as part of a uni-compartmental knee arthroplasty.

### BACKGROUND OF THE INVENTION

Partial knee replacement (UKA) is a well-documented and increasingly used treatment for patients with knee osteoarthritis. Close to 2 million knee replacements are performed annually in OECD countries, and UKA surgery constitutes a growing proportion of these knee replacements.

The performance of a cut in the bone as part of the surgery is typically done manually and causes a risk of cutting at a slightly erroneous angle resulting in an extended cut in the posterior part of the bone. This may cause a later fracture. A fracture related to the prosthesis (periprosthetic fracture) after UKA surgery can be an extremely severe complication. While they rarely occur (estimated 0.4% of UKAs), periprosthetic fractures have a major impact on the outcome for the patients. More invasive revision surgery is needed, resulting in an increased risk of infections and worse knee function.

The association between an extended vertical/sagittal cut in the posterior part of the tibia and periprosthetic fractures is well established. Cadaver investigations with increasingly extended vertical cuts, showed that the forces needed to create a periprosthetic fracture decreased dramatically with the extension of the cuts.

Hence, an improved system for assisting a surgeon during the performance of a cut in a bone of a patient would be advantageous.

### OBJECT OF THE INVENTION

It is an object of the present invention to provide a system for assisting a surgeon during the performance of a cut in a bone of a patient, with which system the risk of cutting too deep into the bone is lower than with known systems.

It is another object of the present invention to provide such a system with which the risk of cutting at a wrong angle in the bone is lower than with known systems.

It is an object of at least some embodiments of the present invention to provide a system which can be used with existing equipment, such as a manually held bone-saw, without the need for modification thereof.

It is a further object of the present invention to provide an alternative to the prior art.

In particular, it may be seen as an object of the present invention to provide a system for assisting a surgeon during the performance of a cut in a bone that solves the above mentioned problems of the prior art.

### SUMMARY OF THE INVENTION

Thus, the above described object and several other objects are intended to be obtained in a first aspect of the invention by providing a system for assisting a surgeon during the performance of a cut in a bone of a patient, the system comprising:
- a first sensor configured to:
   - be arranged on an associated cutting guide placed on the bone in which the cut is to be made, the cutting guide defining a desired cutting orientation,
   - measure the spatial orientation of the cutting guide, and
   - transmit a first output signal representative of the spatial orientation of the cutting guide,
- a second sensor configured to:
   - be arranged on an associated bone-saw to be used for performing the cut,
   - measure the spatial orientation of a blade of the bone-saw, and
   - transmit a second output signal representative of the spatial orientation of the bone-saw,
- a receiver configured to:
   - receive the first and second output signals transmitted by the first and second sensors, respectively,
   - based on the received first and second output signals, determine a difference between the spatial orientations of the blade and the cutting guide and thereby a difference between an actual cutting orientation and a desired cutting orientation of the blade, and
   - present an indication of the difference to the surgeon on at least one angle conformity indicator.

The system will be described with respect to the performance of a sagittal cut, since that is the use for which it was developed. However, it can also be used for cuts in other orientation. In anatomy, the sagittal plane, or longitudinal plane, is an imaginary anatomical plane which divides the body into right and left parts. The plane may be in the centre of the body and split it into two halves (mid-sagittal) or away from the midline and split it into unequal parts (para-sagittal). By "sagittal cut" is meant a cut in a sagittal plane so that if a patient is standing, a "sagittal cut" will be a vertical cut. The system has been developed for use during uni-compartmental knee arthroplasty, but it may be used in any type of cuts where it is important to keep a desired cutting angle. It is particularly advantageous for performing cuts where the surgeon performing the cut has limited or no direct view to the blade of the bone-saw at least during a part of the cutting process.

A cutting guide is a small plate, typically made from metal, configured to be temporarily fixed to the bone by use of pins or screws. With this commonly accepted technique, the cutting guide remains with a stable fixation to the bone, in the desired position as described in the operative technique, throughout the bone cutting procedure. A cutting guide is typically arranged so that the upper surface constitutes a plane which is perpendicular to the cut to be made. Thus, when the bone-saw is kept so that the cutting edge of the blade extends in a direction parallel to the upper surface of the cutting guide, the cut will not continue further into the bone than intended.

The feature that the first sensor is configured to be arranged on an associated cutting guide also includes embodiments in which the first sensor is built-into the cutting guide.

Here and in the following, "bone-saw" is to be understood as any tool that is suitable for the performance of a cut in a bone during a surgery.

The measured spatial orientations of the cutting guide and of the blade of the bone-saw are the actual measures at the time of the measurement. The sampling rate of the first and second sensors should therefore be high enough to ensure that any changes of the spatial orientations are captured fast enough to prevent any accidental cuts outside of the desired cutting orientation provided that the surgeon reacts immediately to any changes of the indication presented on the at least one angle conformity indicator. The term "measure" used here and in the following in relation to the first and second sensors could also be referred to as "detect" or "detect and measure". The term "spatial orientation" means a three-dimensional representation of the roll and pitch of the sensors compared to a given fixed orientation which is typically with the mounting surface of the sensor horizontal and with a height of the sensor aligned with the direction of gravity. However, for other desired cutting orientations, this may vary correspondingly.

The use of a system according to the invention is expected to lower the risk of bone fracture caused by excessive angling during the performance of a cut during knee surgery. Such a fracture has a large impact on the patients experiencing it. Furthermore, a lowering of the risk of making an error will improve the working conditions for the surgeon, since such risk can cause an unpleasant pressure even on experienced surgeons.

At least one of the first and second sensors may comprise a gyroscope. Furthermore, at least one of the first and second sensors may comprise an accelerometer. In presently preferred embodiments, both the first and the second sensors comprise both a gyroscope and an accelerometer. The gyroscope measures the orientation based gravity, and the accelerometer measures the orientation based on acceleration/vibration. They therefore complement each other and at least for some sensors result in a higher precision in the calculation of the angle than if only one type of sensor was used. Such types of sensors are already on the market and ready for use in the system. It is therefore expected that the cost of a system according to the invention will be relatively low compared to the cost of just one knee surgery.

In some embodiments of the invention, at least one of the first and second sensors is configured to be encoded in dependence of an actual application. Hereby the same sensors could be used in multiple types of surgery, by encoding the reference angles and any needed offsets to align the bone-saw with an optimal reference angle for a specific cut to be made.

The first and/or the second output signals may be wirelessly transmitted to the receiver. This will make the handling of the system easier both during the placement of the cutting guide and the sensor thereon and during the performance of the cut. This is e.g. because the mutual arrangement of the sensors and the receiver is not limited by otherwise possible cables and because this removes the risk of damaging a cable. Furthermore, it is advantageous when the sensors are to be used in a sterile operating theatre where cables across the surgical field could cause contamination. The first and/or second signals may e.g. be radiofrequency signal(s) or Bluetooth signal(s).

In some embodiments of the invention, the second sensor is configured to be integrated in the bone-saw. It may e.g. be a permanent integration, possibly made during the manufacturing of the bone-saw, or it may be a replaceable arrangement. It may e.g. be possible to use different second sensors in a given bone-saw, so that the second sensor can be selected for a given application. Alternatively, the second sensor is to be mounted on an outer surface of the bone-saw without being integrated therewith. Such mounting could be by any suitable means such as by a screw-connection or by mutually engaging hatches.

The receiver may be configured to be adjusted with respect to how the indication of the difference should be presented. It may e.g. be possible to change between different types of indication dependent on a surgeon's preference.

The angle conformity indicator may comprise one or more of the following: a display showing a numerical value, at least one light indicator of variable colour, a plurality of light indicators that can be individually lit, and a loudspeaker. When the angle conformity indicator provides a visual indication, it should preferably be arranged within the field of view of the surgeon during the performance of the cut while he can still watch the blade.

The receiver may be configured to send a switch-off signal to the bone-saw when the determined difference exceeds a predefined threshold value. Hereby a larger safety can be built into the system, since the bone-saw can be stopped automatically even if the surgeon does not react as intended in response to the provided indication.

In a second aspect, the invention relates to a method of assisting a surgeon during the performance of a cut in a bone by use of a system according to the first aspect of the invention, the method comprising the following steps:
- placing a cutting guide on the bone in which the cut is to be made,
- arranging the first sensor on the cutting guide or using a cutting guide comprising a built-in first sensor,
- arranging the second sensor on the bone-saw,
- performing the cut while paying attention to the indication of the difference between the spatial orientations of the blade and the desired cutting orientation via the angle conformity indicator, and
- aiming at keeping the difference as close to zero as possible.

The step of arranging the second sensor on the bone-saw may be performed at a previous time than the other method steps. It will e.g. be possible to keep the second sensor on the bone-saw for longer periods of time instead of having to re-arrange it there before each use. Correspondingly, the first sensor may be permanently built into the cutting guide so that the step of arranging the first sensor on the cutting guide is performed at a previous time.

In some embodiments of the invention, the bone-saw is manually handled. This is the use for which the invention was developed, since this is the use where the risk of performing a cut at a wrong angle is highly dependent on the skills of the surgeon.

In alternative embodiments of the invention, the cutting guide and/or the bone-saw is integrated in a robot.

The first and second aspects of the present invention may each be combined with any of the other aspects. These and other aspects of the invention will be apparent from and elucidated with reference to the embodiments described hereinafter.

### BRIEF DESCRIPTION OF THE FIGURES

The system according to the invention as well as possible implementations thereof will now be described in more detail with regard to the accompanying figures. The figures show one way of implementing the present invention and is not to be construed as being limiting to other possible embodiments falling within the scope of the attached claim set.
Figure 1 schematically shows the bones of a knee and a bone-saw for performing a cut in the knee; the figure shows a sagittal cut. Figure 1.a shows the intended angle of the bone-saw and figure 1.b exaggerated shows a wrong cutting angle that will result in too deep a cut.
Figure 2 schematically shows an embodiment of the invention.
Figure 3 schematically shows first and second sensors each provided with a gyroscope and an accelerometer.
Figure 4 is a flow-chart of a method according to the invention.

### DETAILED DESCRIPTION OF AN EMBODIMENT

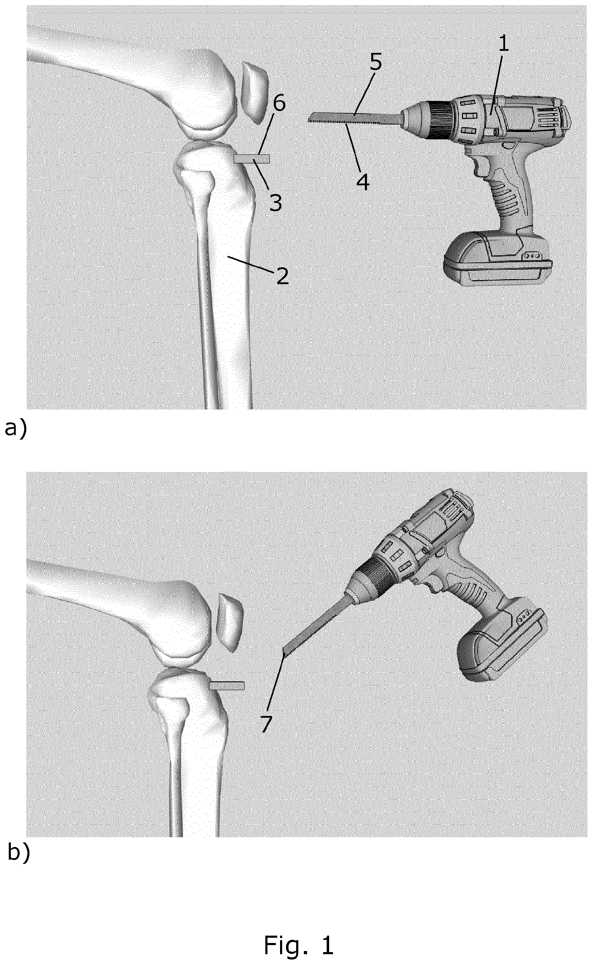

Figure 1 schematically illustrates how it can cause problems when a bone-saw 1 is not held at the correct angle during the performance of a sagittal cut in a bone 2 of a patient. In the illustrated embodiment, the bone 2 is a knee, since the invention has been developed based on a need for improvement in relation to uni-compartmental knee arthroplasty. However, the system can also be used for other types of surgery where a cutting guide is used, and where assistance of the cutting angle is of advantage. Figure 1.a shows a bone 2 on which a cutting guide 3 has been placed in order to guide the surgeon with respect to both cutting depth and cutting angle. The intention is to hold the bone-saw 1 so that the cutting edge 4 of the blade 5 is parallel to a plane defined by the upper surface 6 of the cutting guide 3. Figure 1.b shows in exaggerated view what will happen, if the bone-saw 1 is held at a wrong angle. This means that the cut being made will extend deeper into the bone 2 than planned, because the front end 7 of the blade 5 does not touch the cutting guide 3. Such a too deep cut can unnecessarily weaken the bone 2 and increase the risk of a later fracture. It can also result in other complications which can result from a negative interaction between the bone 2 and an implant (not shown) to be inserted.

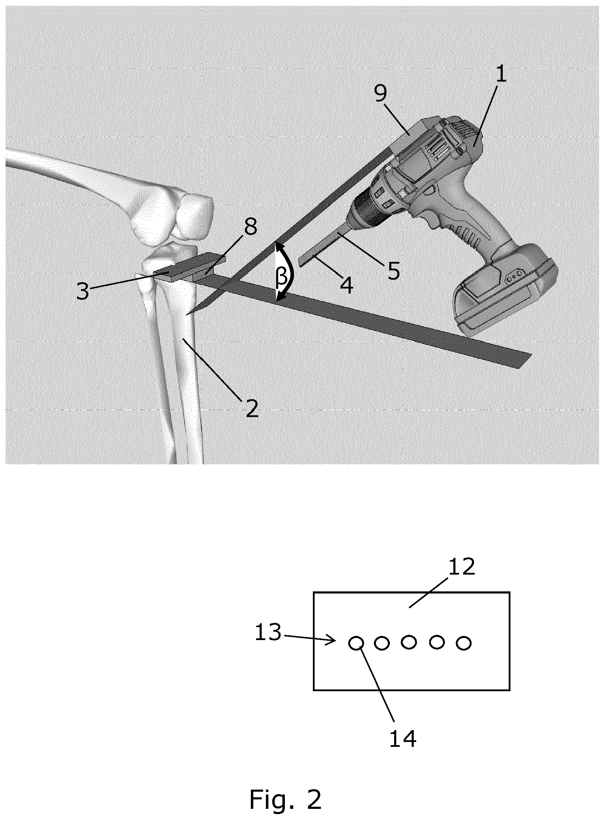

Figure 2 schematically shows a system according to the invention when ready for use for the performance of a sagittal cut. The system would work in the same manner for cuts being performed in other orientations, such as a horizontal cut. A first sensor 8 is arranged on the cutting guide 3 which is placed on the bone 2 in which the sagittal cut is to be made. As explained in relation to figure 1, the cutting guide 3 defines a desired cutting orientation and cutting depth. A second sensor 9 is arranged on an outer surface of the bone-saw 1 to be used for performing the sagittal cut. As mentioned above, the second sensor 9 may alternatively be configured to be integrated in the bone-saw 1. The system has been developed for surgeries in which the bone-saw 1 is manually handled. However, the scope of protection also covers systems in which the cutting guide 3 and/or the bone-saw 1 is integrated in a robot (not shown).

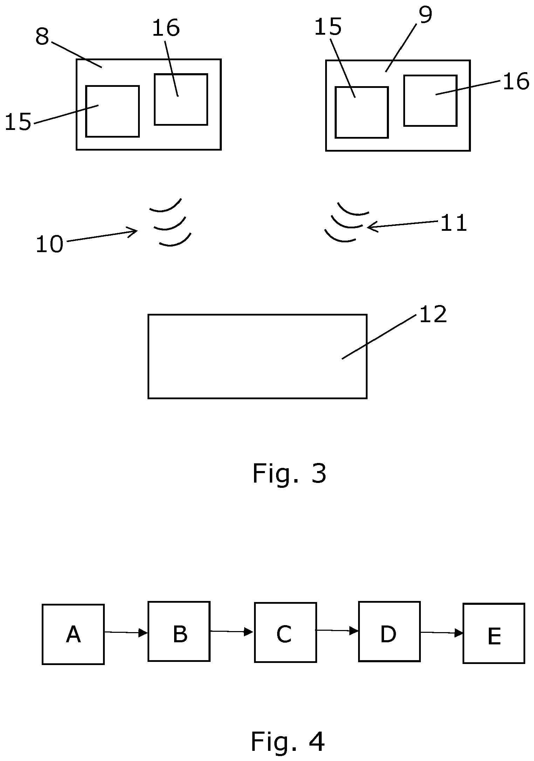

The first sensor 8 is configured to measure the spatial orientation of the cutting guide 3 and transmit a first output signal 10 representative of the spatial orientation of the cutting guide 3; this is shown in figure 3. The second sensor 9 is configured to measure the spatial orientation of the blade 5 of the bone-saw 1 and transmit a second output signal 11 representative of the spatial orientation of the bone-saw 1. The first and second sensors 8,9 may be configured to be encoded in dependence of an actual application. The system further comprises a receiver 12 which is configured to receive the first and second output signals 10,11 transmitted by the first and second sensors 8,9, respectively. Based on the received first and second output signals 10,11, the receiver 12 determines a difference between the spatial orientations of the blade 5 and the cutting guide 3 and thereby a difference between an actual cutting orientation and the desired cutting orientation; this difference is shown as β in figure 2. The receiver 12 then presents an indication of the difference to the surgeon on at least one angle conformity indicator 13. In the figure, the receiver 12 is shown as comprising the angle conformity indicator 13, but it may also be separate units. In the system in figure 2, the angle conformity indicator 13 is a plurality of light indicators 14 that can be individually lit. The light indicators 14 preferably have different colours e.g. so that the middle light indicator is green and indicate that the bone-saw 1 is held at the correct angle, the two neighbouring light indicators are yellow, and the outermost light indicators are red. The surgeon can then visually follow the actual orientation and manually adjust the angle to aim for always having green light. Other types of angle conformity indicators 13 may e.g. be a display showing a numerical value, at least one light indicator of variable colour, or a loudspeaker. More than one type may be used in combination, or it may be possible to switch between different types, e.g. based on the surgeon's personal preference. As mentioned above, the receiver 12 may be configured to send a switch-off signal to the bone-saw 1 when the determined difference β exceeds a predefined threshold value. Hereby the risk of cutting errors can be lowered even further.

In some embodiments of the invention, at least one of the first and second sensors 8,9 comprises a gyroscope 15, and at least one of the first and second sensors 8,9 comprises an accelerometer 16. Figure 3 schematically shows an embodiment in which both sensors 8,9 comprise both a gyroscope 15 and an accelerometer 16. In the embodiment in figure 3, the first and second output signals 10,11 are wirelessly transmitted to the receiver 12, e.g. as radiofrequency or Bluetooth signals.

Figure 4 is a flow-chart of a method according to the second aspect of the invention. The method comprises the following steps:
A: Placing a cutting guide 3 on the bone 2 in which the cut is to be made.
B: Arranging the first sensor 8 on the cutting guide 3 or using a cutting guide 3 comprising a built-in first sensor 8.
C: Arranging the second sensor 9 on the bone-saw 1.
D: Performing the cut while paying attention to the indication of the difference β between the spatial orientations of the blade 5 and the desired cutting orientation via the angle conformity indicator 13.
E: Aiming at keeping the difference β as close to zero as possible.
As mentioned above, step B may be performed at a previous time than the other method steps. It will e.g. be possible to keep the second sensor 9 on the bone-saw 1 for longer periods instead of having to re-arrange it there before each use. Steps D and E will in practise be performed simultaneously.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. System for assisting a surgeon during the performance of a cut in a bone (2) of a patient, the system comprising:
- a first sensor (8) configured to:
• be arranged on an associated cutting guide (3) placed on the bone (2) in which the cut is to be made, the cutting guide (3) defining a desired cutting orientation,
• measure the spatial orientation of the cutting guide (3), and
• transmit a first output signal (10) representative of the spatial orientation of the cutting guide (3),
- a second sensor (9) configured to:
• be arranged on an associated bone-saw (1) to be used for performing the cut,
• measure the spatial orientation of a blade (5) of the bone-saw (1), and
• transmit a second output signal (11) representative of the spatial orientation of the bone-saw (1),
- a receiver (12) configured to:
• receive the first and second output signals (10,11) transmitted by the first and second sensors (8,9), respectively,
• based on the received first and second output signals (10,11), determine a difference (β) between the spatial orientations of the blade (5) and the cutting guide (3) and thereby a difference between an actual cutting orientation and the desired cutting orientation, and
• present an indication of the difference to the surgeon on at least one angle conformity indicator (13).

2. System according to claim 1, wherein at least one of the first and second sensors (8,9) comprises a gyroscope (15).

3. System according to claim 1 or 2, wherein at least one of the first and second sensors (8,9) comprises an accelerometer (16).

4. System according to any of the preceding claims, wherein at least one of the first and second sensors (8,9) is configured to be encoded in dependence of an actual application.

5. System according to any of the preceding claims, wherein the first and/or the second output signals (10,11) is wirelessly transmitted to the receiver (12), such as wherein the first and/or second signals (10,11) is radiofrequency signal(s).

6. System according to any of the preceding claims, wherein the second sensor (9) is configured to be integrated in the bone-saw (1).

7. System according to any of the preceding claims, wherein the receiver (12) is configured to be adjusted with respect to how the indication of the difference (β) should be presented.

8. System according to any of the preceding claims, wherein the angle conformity indicator (13) comprises one or more of the following: a display showing a numerical value, at least one light indicator of variable colour, a plurality of light indicators (14) that can be individually lit, and a loudspeaker.

9. System according to any of the preceding claims, wherein the receiver (12) is configured to send a switch-off signal to the bone-saw (1) when the determined difference (β) exceeds a predefined threshold value.

10. Method of assisting a surgeon during the performance of a cut in a bone (2) by use of a system according to any of the preceding claims, the method comprising the following steps:
- placing a cutting guide (3) on the bone (2) in which the cut is to be made,
- arranging the first sensor (8) on the cutting guide (3) or using a cutting guide (3) comprising a built-in first sensor (8),
- arranging the second sensor (9) on the bone-saw (1),
- performing the cut while paying attention to the indication of the difference (β) between the spatial orientations of the blade (5) and the desired cutting orientation via the angle conformity indicator (13), and
- aiming at keeping the difference (β) as close to zero as possible.

11. Method according to claim 10, wherein the bone-saw (1) is manually handled.

12. Method according to claim 10, wherein the cutting guide (3) and/or the bone-saw (1) is integrated in a robot.
